# EUROPEAN PATENT APPLICATION

(11) **EP 1 736 189 A1**
(43) Date of publication of application: **27.12.2006**
(21) Application number: 05727022.5
(22) Date of filing: 23.03.2005
(51) Int. Cl.: A61M 5/00, A61M 5/145

(54) **MEDICAL LIQUID INJECTION SYSTEM**

(30) Priority: 05.04.2004 JP 2004110853
(71) Applicant: Nemoto Kyorindo Co., Ltd., Tokyo 113-0033 (JP)
(72) Inventor: SAITOH, Yasufumi, c/o Nemoto Kyorindo Co., Ltd., Tokyo 1130033 (JP); MASUDA, Kazumasa, c/o Nemoto Kyorindo Co., Ltd., Tokyo 1130033 (JP)
(74) Representative: Cabinet Plasseraud
(86) International application number: PCT/JP2005/005216
(87) International publication number: WO 2005/097232

(57) **Abstract**

A liquid injection system which can be checked that that a liquid syringe is appropriately mounted on a chemical liquid injector and the liquid syringe is filled with a liquid at a time. Pattern 400 appearing as a predetermined shape when it is viewed from above through liquid syringe 200 filled with transparent liquid L is divided between a lower surface of liquid syringe 200 and an upper surface of chemical liquid injector 100. When liquid syringe 200 is appropriately held by chemical liquid injector 100, the pattern on the lower surface of liquid syringe 200 and the pattern on the upper surface of chemical liquid injector 100 are properly combined. If liquid syringe 200 is filled with a transparent liquid in this state, the combined pattern is viewed as the predetermined shape from above liquid syringe 200. The shape can be seen to check that the liquid syringe 200 is appropriately held and filled with liquid L at a time.

## Description

### Technical Field

The present invention relates to a chemical liquid injection system which moves a cylinder member and a piston member of a liquid syringe relatively by means of a chemical liquid injector to inject a liquid into a patient, and more particularly, to a chemical liquid injection system in which a liquid syringe is mounted on a chemical liquid injector via a cylinder adapter.

### Background Art

Presently available apparatuses for capturing diagnostic images of patients include CT (Computed Tomography) scanners, MRI (Magnetic Resonance Imaging) apparatuses, PET (Positron Emission Tomography) apparatuses, SPECT (Single Photon Emission Computed Tomography) apparatuses, ultrasonic diagnostic apparatuses, angiography apparatuses, MRA (MR angiography) apparatuses and the like.

When the abovementioned apparatuses are used, a liquid such as a contrast medium or physiological saline may be injected into a patient. Chemical liquid injectors for automatically performing the injection have been put into practical use. Such a chemical liquid injector has an injection head on which a liquid syringe is removably mounted.

The liquid syringe has a tubular cylinder member which is filled with a liquid. A tubular piston member is slidably inserted into the cylinder member. Typically, the cylinder member has an annular cylinder flange formed in the outer circumference of the trailing end and the piston member has an annular piston flange formed in the outer circumference of the trailing end.

When the chemical liquid injector is used, the cylinder member of the liquid syringe filled with a liquid is connected to a patient via an extension tube and the liquid syringe is mounted on the injection head of the chemical liquid injector. In a typical chemical liquid injector, the injection head has a concave portion formed in its upper surface in a shape fitting the cylinder member and the cylinder flange of the liquid syringe. The cylinder member and the cylinder flange can be set in the concave portion to hold the liquid syringe.

The chemical liquid injector holds the piston flange by a piston driving mechanism independently of the cylinder member, and the piston member is slid by the piston driving member. In this manner, the liquid can be injected into the patient from the liquid syringe, and as required, the liquid can be sucked into the liquid syringe from a liquid tank.

In the chemical liquid injector as described above, the concave portion of the injection head is generally formed to fit the cylinder member of the liquid syringe of the maximum size in order to accommodate a plurality of types of liquid syringes having various shapes. A liquid syringe of a size other than the maximum size is set in the concave portion of the injection head after a dedicated cylinder adapter is attached to a cylinder member thereof.

In general, the cylinder adapter described above also has a concave portion in its upper surface in a shape fitting the cylinder member and the cylinder flange to be mounted, similarly to the injection head. The cylinder member and the cylinder flange of the liquid syringe are held in the concave portion. The cylinder adapter has a lower surface formed to have an outer shape similar to that of the cylinder member and the cylinder flange of the liquid syringe of the maximum size and is set in the concave portion of the injection head.

Chemical liquid injectors of the type as described above have been devised and filed for patent by the applicant of the present application and the like (see, for example, patent documents 1 and 2 below).
Patent document 1: Japanese laid-open patent publication No. 2002-11096;
Patent document 2: Japanese laid-open patent publication No. 2002-102343.

### Disclosure of the Invention

### Subject to Be Solved by the Invention

In the chemical liquid injector as described above, the liquid syringe is held by directly mounting the cylinder member in the concave portion of the injection head or by mounting the cylinder adapter, with the cylinder member set in its concave portion, in the concave portion of the injection head. However, it is difficult to hold the liquid syringe securely only by the setting in the concave portion and thus the liquid syringe may be mounted improperly and come off the injection head during injection operation.

To solve the problem, some measures have been taken. For example, concave portions are formed on both sides on the left and right of a cylinder flange of a liquid syringe, a pair of elastic arms which can deform elastically leftward and rightward are placed on an injection head or the cylinder flange, and the concave portions of the cylinder flange on the left and right are elastically held by the pair of elastic arms.

As another example, a cylinder flange of a liquid syringe has both sides on the top and bottom formed in flat shape, a pair of clamp mechanisms openable and closeable in the left-and-right direction are placed on an injection head or the cylinder flange, and the cylinder flange is held by the pair of clamp mechanisms on the left and right.

In these mechanisms, the up and down directions of the cylinder member are defined, and the liquid syringe needs to be set in a cylinder holding mechanism with the up and down directions appropriately oriented. When the liquid syringe is set in the cylinder holding mechanism with the up and down directions of the cylinder member oriented inappropriately, the elastic arms do not fit into the concave portions of the cylinder flange or the clamp mechanisms do not properly secure the cylinder flange, thereby resulting in poor holding of the liquid syringe.

A typical liquid syringe has a cylinder member formed of colorless and transparent glass or resin to allow an operator to easily check filling of a liquid. In some cases, a liquid such as physiological saline or a contrast medium to fill the liquid syringe is also colorless and transparent.

In such a case, the filling of the liquid is checked as a result of refraction of a background scene visually recognized through the cylinder member. It may be inadvertently determined that the liquid syringe is filled with the liquid even when it is not filled. Then, the liquid syringe filled with air may be mounted on a chemical liquid injector and connected to a blood vessel of a patient, which can cause medical malpractice of injection of the air into the blood vessel of the patient.

The present invention has been made in view of the abovementioned problems, and it is an object thereof to provide a chemical liquid injection system which can be checked that a liquid syringe is appropriately mounted on a chemical liquid injector and the liquid syringe is filled with a liquid at a time.

### Means to Solve the Subject

According to a first aspect of the present invention, a chemical liquid injection system comprises a chemical liquid injector and a liquid syringe. The liquid syringe comprises a transparent cylinder member having at least a position defined as a lower surface, and a piston member slidably inserted into the cylinder member. The chemical liquid injector comprises a cylinder holding mechanism removably holding the cylinder member of the liquid syringe mounted from above in a state that the lower surface is positioned downward, and a piston driving mechanism relatively moving the piston member to the held cylinder member of the liquid syringe. A pattern appearing as a predetermined shape when it is viewed from above through the cylinder member filled with a transparent liquid is divided between a lower surface of the cylinder member and an upper surface of the cylinder holding mechanism. When the cylinder member is appropriately held by the cylinder holding mechanism, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member.

According to a second aspect of the present invention, a chemical liquid injection system comprises a chemical liquid injector, liquid syringes of various sizes, and a cylinder adapter of at least one type. The cylinder adapter is formed for each of the liquid syringes of sizes other than the maximum size and removably holds the cylinder member mounted from above in a state that the lower surface is positioned downward. A cylinder holding mechanism of the chemical liquid injector removably holds the cylinder member of the liquid syringe of the maximum size mounted from above and the cylinder member of the liquid syringe of a size other than the maximum size mounted from above via the cylinder adapter in a state that the lower surface is positioned downward. A pattern appearing as a predetermined shape when it is viewed from above through the cylinder member filled with a transparent liquid is divided between a lower surface of the cylinder member and an upper surface of the cylinder holding mechanism. The cylinder adapter is formed transparently. When the cylinder member is appropriately held by the cylinder holding mechanism via the cylinder adapter, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member.

According to a third aspect of the present invention, a chemical liquid injection system comprises a chemical liquid injector, liquid syringes of various sizes, and a cylinder adapter of at least one type. The cylinder adapter is formed non-transparently. A pattern appearing as a predetermined shape when it is viewed from above through the cylinder member filled with a transparent liquid is divided between a lower surface of the cylinder member of the liquid syringe of the maximum size and an upper surface of the cylinder holding mechanism, and a pattern appearing as a predetermined shape when it is viewed from above through the cylinder member filled with a transparent liquid is divided between a lower surface of the cylinder member of the liquid syringe of a size other than the maximum size and an upper surface of the corresponding cylinder adapter. When the cylinder member of the liquid syringe of the maximum size is appropriately held by the cylinder holding mechanism, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member. When the cylinder member of the liquid syringe of a size other than the maximum size is appropriately held by the cylinder adapter, the pattern on the lower surface of the cylinder member and the pattern on the upper surface of the cylinder adapter are properly combined. If the cylinder member is filled with a transparent liquid in this state, the combined pattern is viewed as the predetermined shape from above the cylinder member.

According to a fourth aspect of the present invention, a chemical liquid injection system comprises a chemical liquid injector, liquid syringes of various sizes, and a cylinder adapter of at least one type. The cylinder adapter is formed transparently. A pattern appearing as a predetermined shape when it is viewed from above through the cylinder member filled with a transparent liquid is divided among a lower surface of the cylinder member of the liquid syringe of a size other than the maximum size, the corresponding cylinder adapter, and an upper surface of the cylinder holding mechanism. The pattern formed by combining the pattern on the lower surface of the cylinder member of the liquid syringe of the size other than the maximum size with the pattern on the corresponding cylinder adapter is provided for a lower surface of the cylinder member of the liquid syringe of the maximum size. When the cylinder member of the liquid syringe of the maximum size is appropriately held by the cylinder holding mechanism, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member. When the cylinder member of the liquid syringe of a size other than the maximum size is appropriately held by the cylinder holding mechanism via the cylinder adapter, the pattern on the lower surface of the cylinder member, the pattern on the cylinder adapter, and the pattern on the upper surface of the cylinder holding mechanism are properly combined. If the cylinder member is filled with a transparent liquid in this state, the combined pattern is viewed as the predetermined shape from above the cylinder member.

While the directions of up and down are defined herein, these directions are defined for convenience in easily explaining the relative relationship between the directions, and the definition does not limit any direction in manufacture or use when the present invention is implemented.

### Effect of the Invention

In the chemical liquid injection system according to the first aspect of the present invention, the pattern appearing as a predetermined shape when it is viewed from above through the cylinder member filled with a transparent liquid is divided between the lower surface of the cylinder member and the upper surface of the cylinder holding mechanism. When the cylinder member is appropriately held by the cylinder holding mechanism, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member. The shape can be seen to check that the liquid syringe is appropriately held by the cylinder holding mechanism and that the cylinder member is filled with the liquid at a time.

In the chemical liquid injection system according to the second aspect of the present invention, the pattern appearing as a predetermined shape when it is viewed from above through the cylinder member filled with a transparent liquid is divided between the lower surface of the cylinder member and the upper surface of the cylinder holding mechanism. The cylinder adapter is formed transparently. When the cylinder member is appropriately held by the cylinder holding mechanism via the cylinder adapter, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member. The shape can be seen to check that the liquid syringe is appropriately held by the cylinder holding mechanism and that the cylinder member is filled with the liquid at a time.

In the chemical liquid injection system according to the third aspect of the present invention, the cylinder adapter is formed non-transparently. The pattern appearing as a predetermined shape when it is viewed from above through the cylinder member filled with a transparent liquid is divided between the lower surface of the cylinder member of the liquid syringe of the maximum size and the upper surface of the cylinder holding mechanism. The pattern appearing as a predetermined shape when it is viewed from above through the cylinder member filled with a transparent liquid is divided between the lower surface of the cylinder member of the liquid syringe of a size other than the maximum size and the upper surface of the corresponding cylinder adapter. When the cylinder member of the liquid syringe of the maximum size is appropriately held by the cylinder holding mechanism, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member. The shape can be seen to check that the liquid syringe of the maximum size is appropriately held by the cylinder holding mechanism and that the cylinder member is filled with the liquid at a time. When the cylinder member of the liquid syringe of a size other than the maximum size is appropriately held by the cylinder adapter, the pattern on the lower surface of the cylinder member and the pattern on the upper surface of the cylinder adapter are properly combined. If the cylinder member is filled with a transparent liquid in this state, the combined pattern is viewed as the predetermined shape from above the cylinder member. The shape can be seen to check that the liquid syringe is appropriately held by the cylinder adapter and that the cylinder member is filled with the liquid at a time.

In the chemical liquid injection system according to the fourth aspect of the present invention, the cylinder adapter is formed transparently. The pattern appearing as a predetermined shape when it is viewed from above through the cylinder member filled with a transparent liquid is divided among the lower surface of the cylinder member of the liquid syringe of a size other than the maximum size, the corresponding cylinder adapter, and the upper surface of the cylinder holding mechanism. The pattern formed by combining the pattern on the lower surface of the cylinder member of the liquid syringe of the size other than the maximum size with the pattern on the corresponding cylinder adapter is provided for the lower surface of the cylinder member of the liquid syringe of the maximum size. When the cylinder member of the liquid syringe of the maximum size is appropriately held by the cylinder holding mechanism, the pattern on the lower surface of the cylinder member and the pattern on the upper surface of the cylinder holding mechanism are properly combined. If the cylinder member is filled with a transparent liquid in this state, the combined pattern is viewed as the predetermined shape from above the cylinder member. The shape can be seen to check that the liquid syringe of the maximum size is appropriately held by the cylinder holding mechanism and that the cylinder member is filled with the liquid at a time. When the cylinder member of the liquid syringe of a size other than the maximum size is appropriately held by the cylinder holding mechanism via the cylinder adapter, the pattern on the lower surface of the cylinder member, the pattern on the cylinder adapter, and the pattern on the upper surface of the cylinder holding mechanism are properly combined. If the cylinder member is filled with a transparent liquid in this state, the combined pattern is viewed as the predetermined shape from above the cylinder member. The shape can be seen to check that the liquid syringe is appropriately held by the cylinder holding mechanism via the cylinder adapter and that the cylinder member is filled with the liquid at a time.

### Brief Description of the Drawings

Figs. 1 are plan views showing a liquid syringe mounted on a chemical liquid injector of a chemical liquid injection system according to an embodiment of the present invention.
Fig. 2 is a perspective view showing the outer appearance of the chemical liquid injection system.
Fig. 3 is a perspective view showing the outer appearance of the chemical liquid injector.
Figs. 4 are perspective views showing how to mount and remove a liquid syringe on an injection head of the chemical liquid injector.
Figs. 5 are rear views showing the relationship between a cylinder flange of a liquid syringe and the main part of a cylinder holding mechanism.
Fig. 6 is a plan view showing the main portions of the injection head.
Figs. 7 are bottom views and plan views showing the outer appearance of the liquid syringe.
Figs. 8 are perspective views showing how to mount and remove a liquid syringe on an injection head of a chemical liquid injection system of a first modification.
Fig. 9 is a plan view showing a liquid syringe of a chemical liquid injection system of a second modification.
Fig. 10 is a plan view showing a cylinder adapter of the chemical liquid injection system of the second modification.
Fig. 11 is a plan view showing the liquid syringe mounted on a chemical liquid injector via the cylinder adapter in the chemical liquid injection system of the second modification.
Figs. 12 are plan views showing a liquid syringe mounted on a chemical liquid injector via a cylinder adapter in a chemical liquid injection system of a third modification.
Figs. 13 are plan views showing a liquid syringe mounted on a cylinder adapter in a chemical liquid injection system of a fourth modification.
Fig. 14 is a plan view showing a liquid syringe mounted on a chemical liquid injector in a chemical liquid injection system of a fifth modification.

### Description of Reference Numerals

- 100: CHEMICAL LIQUID INJECTOR
- 114: PISTON DRIVING MECHANISM
- 120: CYLINDER HOLDING MECHANISM
- 200: LIQUID SYRINGE
- 210: CYLINDER MEMBER
- 220: PISTON MEMBER
- 400, 401, 411, 412, 402, 420: PATTERN
- 500, 510: CYLINDER ADAPTER
- 1000, 1300: CHEMICAL LIQUID INJECTION SYSTEM
- L: LIQUID

### Best Mode for Carrying the Invention

### (Configuration of Embodiment)

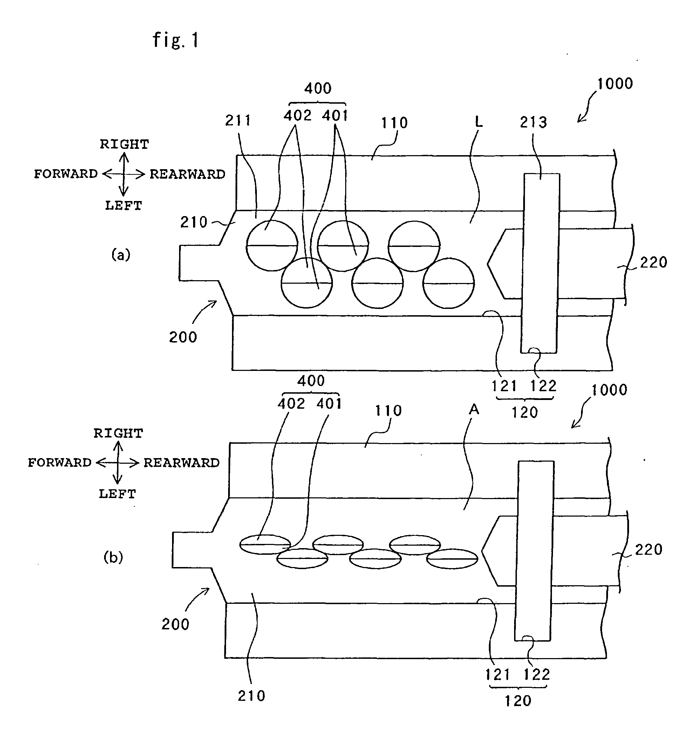

An embodiment of the present invention will hereinafter be described with reference to the drawings. As shown in Figs. 1 to 3, chemical liquid injection system 1000 of the embodiment comprises chemical liquid injector 100, liquid syringe 200, and MRI apparatus 300 serving as a diagnostic imaging apparatus. Chemical liquid injector 100 injects liquid L such as a contrast medium and physiological saline from liquid syringe 200 into a patient (not shown) whose diagnostic images are to be taken by MRI apparatus 300.

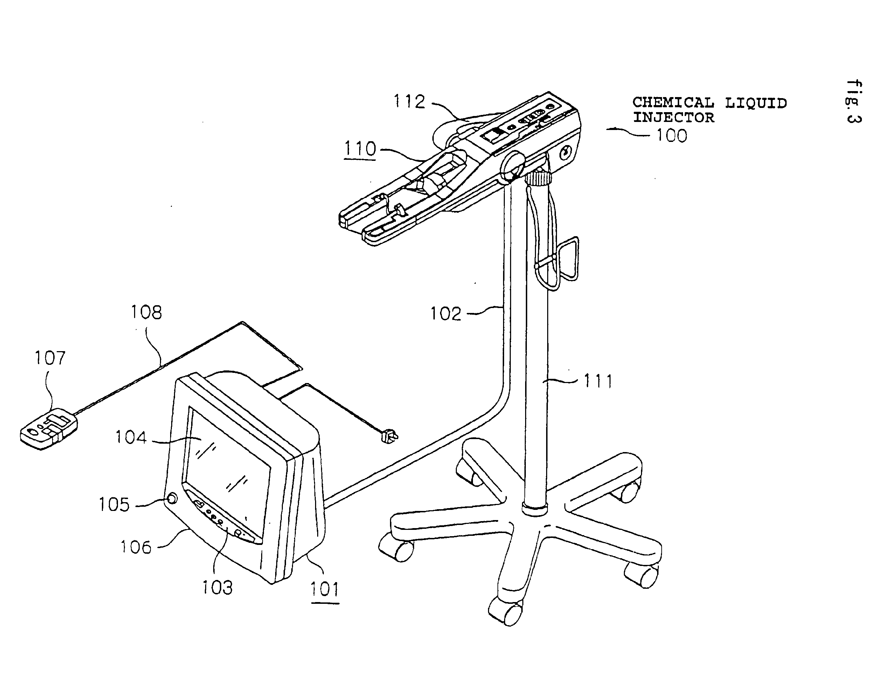

As shown in Fig. 3, MRI apparatus 300 includes diagnostic imaging unit 301 serving as an imaging execution mechanism and imaging control unit 302. Diagnostic imaging unit 301 and imaging control unit 302 are wire-connected through communication network 311. Diagnostic imaging unit 301 captures diagnostic images of the patient, imaging control unit 302 controls the operation of diagnostic imaging unit 301.

As shown in Fig. 1, liquid syringe 200 comprises cylinder member 210 and piston member 220 wherein piston member 220 is slidably inserted into cylinder member 210. Cylinder member 210 has hollow tubular cylinder body 211 having conduit 212 formed at the closed leading end surface.

The trailing end of cylinder body 211 of cylinder member 210 is opened and piston member 220 is inserted from the opening into the interior of cylinder body 211. Cylinder member 210 has cylinder flange 213 formed on the outer circumference of the trailing end, and piston member 220 has piston flange 221 formed on the outer circumference of the trailing end. Cylinder member 210 is formed of transparent glass or resin and is filled with transparent liquid L such as a contrast medium and physiological saline.

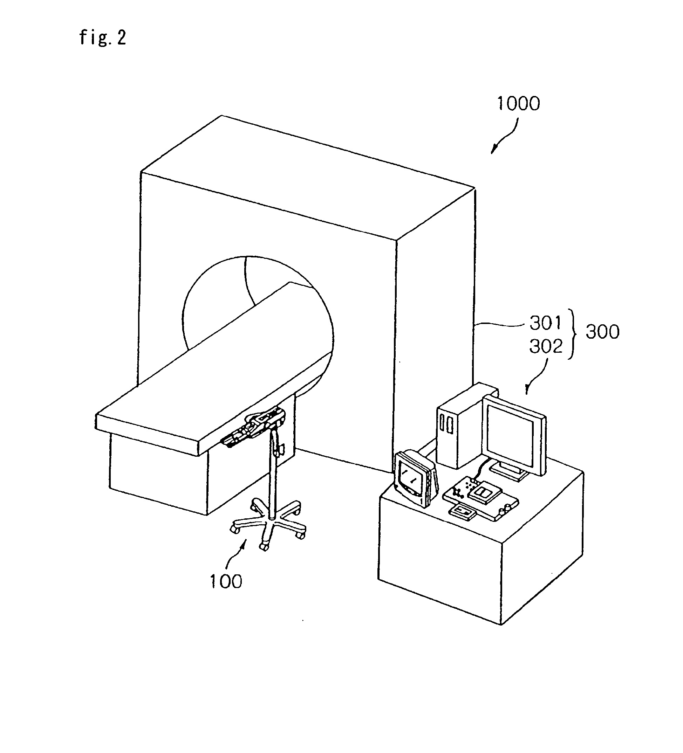

As shown in Fig. 2, chemical liquid injector 100 of the embodiment has injection control unit 101 and injection head 110 serving as an injection head constructed as separate components. Injection control unit 101 and injection head 110 are wire-connected through communication cable 102. Injection head 110 drives liquid syringe 200 mounted thereon to inject liquid L into the patient, and injection control unit 101 controls the operation of injection head 110.

Thus, injection control unit 101 contains a microcomputer (not shown) and is wire-connected to imaging control unit 302 of MRI apparatus 300. As shown in Fig. 2, injection control unit 101 has operation panel 103, touch panel 104 serving as a display panel, speaker unit 105 and the like, all of which are disposed on the front face of unit housing 106. Injection control unit 101 is wire-connected to controller unit 107 as a separate component through connector 108.

Injection head 110 is movably attached to the top end of caster stand 111 by movable arm 112. As shown in Fig. 1, head body 113 has semi-cylindrical concave groove 121 in the upper surface of the forward section in a shape fitting the outer shape of cylinder member 210 of liquid syringe 200.

Flange holding mechanism 122 is formed in concave groove 121 for removably holding cylinder flange 213 of liquid syringe 200. Concave groove 121 of head body 113 and flange holding mechanism 122 constitute cylinder holding mechanism 120 for removably holding cylinder member 210 of liquid syringe 200.

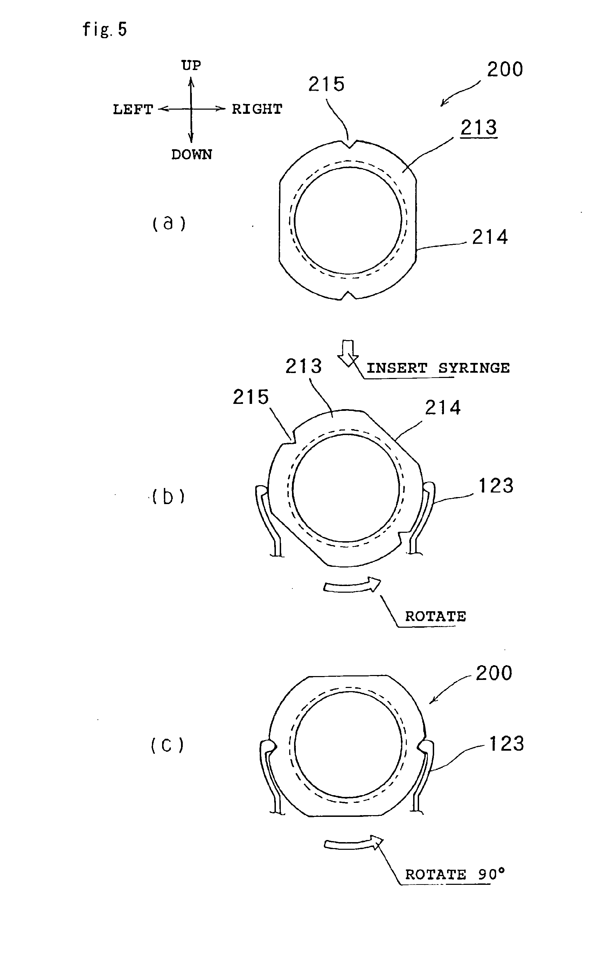

More specifically, as shown in Figs. 5, cylinder flange 213 of liquid syringe 200 is formed to have two parallel sides, that is, two flat portions 214 opposite to each other in the outer circumference. Concave portions 215 are formed at two positions of the sides orthogonal to flat portions 214. In liquid syringe 200 of the embodiment, one of two flat portions 214 of cylinder flange 213 is defined as the position defined as the lower surface of cylinder member 210, while the other is defined as the position defined as the upper surface thereof.

On the other hand, flange holding mechanism 122 of injection head 110 has a pair of elastic arms 123 on the left and right. Elastic arms 123 are formed to have particular shapes at particular positions such that cylinder flange 213 with flat portions 214 located on the left and right can be freely moved upward and downward and concave portions 215 of cylinder flange 213 are held elastically on the left and right.

Liquid syringe 200 is inserted from above into cylinder holding mechanism 120 of injection head 110 with flat portions 214 located on the left and right as described above, cylinder body 211 is caused to contact concave groove 121. In this state, cylinder member 210 is rotated such that the flat portion defined as the position which should be located at the lower surface faces downward. Then, paired elastic arms 123 on the left and right elastically engage with concave portions 215 of cylinder flange 213 on the left and right ends.

Piston driving mechanism 114 comprising a driving motor, a screw mechanism and the like is placed in the rearward section of concave groove 121 of injection head 110. Piston driving mechanism 114 holds and slides piston flange 221 forward and rearward. Piston driving mechanism 114 has an ultrasonic motor (not shown) as a driving motor which is free from generation of magnetic field. The ultrasonic motor is made of nonmagnetic metal such as phosphor bronze alloy (Cu + Sn +P), titanium alloy (Ti-6A1-4V), and magnesium alloy (Mg+Al+Zn).

Chemical liquid injection system 1000 of the embodiment has chemical liquid injector 100 and liquid syringe 200 as described above, and transparent cylinder member 210 of liquid syringe 200 has the specified position defined as the lower surface. Liquid syringe 200 is mounted on injection head 110 of chemical liquid injector 100 in a state that the lower surface is positioned downward. As shown in Fig. 1(a), in chemical liquid injection system 1000 of the embodiment, patterns 400 which appear as predetermined shapes when they are seen from above through cylinder member 210 filled with transparent liquid L are divided between the lower surface of cylinder member 210 and the upper surface of cylinder holding mechanism 120.

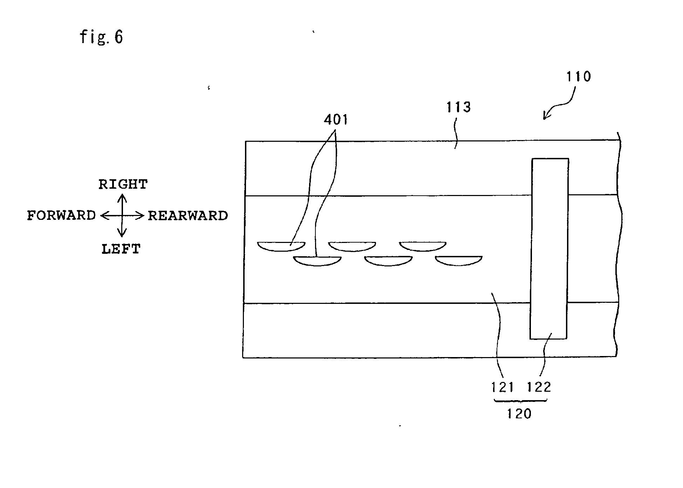

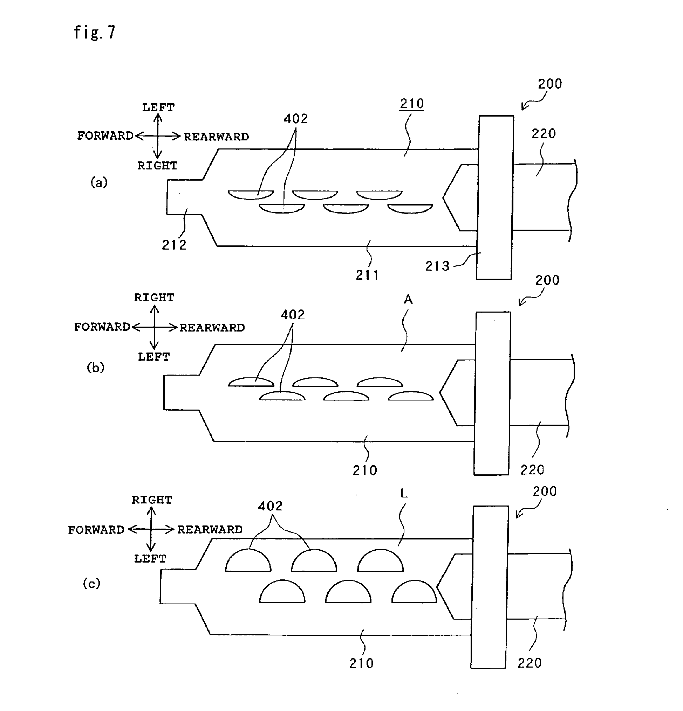

More specifically, as shown in Fig. 6, in chemical liquid injector 100, a plurality of patterns 401 each representing a left half of an ellipse elongated in the forward-and-rearward direction are provided in a predetermined color for the upper surface of concave groove 121 of injection head 110. In liquid syringe 200, as shown in Fig. 7(a), a plurality of patterns 402 each representing a right half of an ellipse elongated in the forward-and-rearward direction are provided in a predetermined color at the position of the lower surface of cylinder member 210 which should be located at the lower surface.

Since cylinder member 210 is formed in a hollow cylindrical shape, it serves as a cylindrical lens which refracts light only in the left-and-right direction if it is filled with transparent liquid L. Each pattern 402 viewed from above through cylinder member 210 appears as a semiellipse elongated in the forward-and-rearward direction without any change if cylinder member 210 is filled with air A as shown in Fig. 7(b). If cylinder member 210 is filled with transparent liquid L, each pattern 402 appears as a semicircle as show in Fig. 7(c).

Patterns 401 and 402 of chemical liquid injector 100 and liquid syringe 200, respectively, are provided at a plurality of positions where they match. When cylinder member 210 is appropriately mounted on injection head 110 as shown in Fig. 1, patterns 401 and 402 are combined into patterns 400.

Thus, patterns 400 viewed from above through cylinder member 210 appropriately mounted on injection head 110 appear as the ellipses elongated in the forward-and-rearward direction as shown in Fig. 1(b) if cylinder member 210 is filled with air A. If cylinder member 210 is filled with transparent liquid L, they appear as perfect circles which are predetermined shapes as shown in Fig. 1(a).

Patterns 401 and 402 described above are provided for chemical liquid injector 100 and liquid syringe 200, respectively, by painting or labeling, for example. They are provided in a color which can be visually recognized favorably from above through cylinder member 210 filled with the liquid and mounted on injection head 110.

### (Operation of Embodiment)

To use chemical liquid injection system 1000 of the embodiment with the abovementioned structure, for example, an operator connects liquid syringe 200 to a blood vessel of a patient in diagnostic imaging unit 301 of MRI apparatus 300 via an extension tube (not shown) and mounts liquid syringe 200 on chemical liquid injector 100.

Piston member 220 of liquid syringe 200 is pressed into cylinder member 210 by piston driving mechanism 114 of chemical liquid injector 100 to inject liquid L such as a contrast medium into the patient from cylinder member 210. Thus, diagnostic images of the patient injected with liquid L are captured by MRI apparatus 300.

If liquid syringe 200 is not properly mounted on chemical liquid injector 100, liquid syringe 200 may come off chemical liquid injector 100, for example when piston member 220 is pressed into cylinder member 210. If liquid syringe 200 filled with air A, rather than liquid L, is used since it was used once or for any other reasons, medical malpractice may occur such as injection of air A into the blood vessel of the patient.

In chemical liquid injection system 1000 of the embodiment, however, patterns 401 and 402 are provided for chemical liquid injector 100 and liquid syringe 200, respectively. Thus, it can be checked at a time that liquid syringe 200 is appropriately mounted on chemical liquid injector 100 and liquid syringe 200 is filled with liquid L.

More specifically, when liquid syringe 200 is mounted on chemical liquid injector 100 as described above, liquid syringe 200 is inserted into cylinder holding mechanism 120 of injection head 110 from above in a state that flat portions 214 of cylinder flange 213 are positioned on the left and right as shown in Fig. 5. Then, cylinder body 211 is caused to contact concave groove 121, and in this state, cylinder member 210 is rotated to face the lower surface downward. Concave portions 215 of cylinder flange 213 located on the left and right are elastically held by paired elastic arms 123 on the left and right.

When liquid syringe 200 is appropriately mounted on injection head 110 in this manner, patterns 401 provided for the upper surface of concave groove 121 of injection head 110 and patterns 402 provided for the lower surface of cylinder member 210 are combined into patterns 400 as shown in Fig. 1.

Tubular cylinder member 210 serves as a cylindrical lens which refracts light only in the left-and-right direction if it is filled with transparent liquid L. Patterns 400 viewed from above through cylinder member 210 appear as the ellipses elongated in the forward-and-rearward direction as shown in Fig. 1(b) if cylinder member 210 is filled with air A. If cylinder member 210 is filled with transparent liquid L, they appear as the perfect circles as shown in Fig. 1(a).

Consequently, in chemical liquid injection system 1000 of the embodiment, when liquid syringe 200 is mounted on chemical liquid injector 100, the correct combination of patterns 401, 402 can be seen to check whether or not liquid syringe 200 is appropriately mounted on injection head 110 at a glance. At the same time, the perfect circles of combined patterns 400 can also be seen to check whether liquid syringe 200 is filled with liquid L or air A at a glance.

Thus, in chemical liquid injection system 1000 of the embodiment, it can be checked that liquid syringe 200 is appropriately held by cylinder holding mechanism 120 and that cylinder member 210 is filled with liquid L at a time. This can prevent liquid syringe 200 from coming off chemical liquid injector 100 during injection operation and medical malpractice of injection of air A into the blood vessel of the patient.

### (Modifications of Embodiment)

The present invention is not in any way limited to the abovementioned embodiment, but various changes and modifications may be made therein without departing from the scope of the invention. For example, in the above embodiment, chemical liquid injector 100 is used near MRI apparatus 300. Alternatively, chemical liquid injector 100 may be used near a CT scanner or an angiography apparatus.

In the above embodiment, patterns 401, 402 are formed with the printing or the like in chemical liquid injector 100 and liquid syringe 200. For example, patterns 401, 402 may be represented on adhesive labels (not shown) which are distributed to a medical facility, thereby providing patterns 401, 402 for an existing chemical liquid injector and an existing liquid syringe in the medical facility.

In the above embodiment, patterns 401, 402 are provided in the predetermined color for chemical liquid injector 100 and liquid syringe 200. For example, patterns 402 may be formed in liquid syringe 200 with a paint which shows a predetermined color at a predetermined temperature. In general, liquid L such as a contrast medium and physiological saline to be injected into a patient in large amount needs to be heated to a temperature equivalent to body temperature previously.

For example, patterns 402 may be formed in liquid syringe 200 with a paint which shows color only when liquid L is at an appropriate temperature and shows no color when liquid L is not at the appropriate temperature, thereby allowing to check whether or not liquid L is at the appropriate temperature by the color of patterns 402. In this case, it can be checked whether or not liquid syringe 200 is properly held by cylinder holding mechanism 120, cylinder member 210 is filled with liquid L, and liquid L is at the appropriate temperature at a time.

In the above embodiment, patterns 400 are divided into patterns 401, 402 in terms of the shapes and arrangements. Such patterns may be divided in terms of color. For example, a green pattern of a predetermined shape may be provided in a predetermined arrangement for chemical liquid injector 100 and a red pattern of the same shape may be provided in the same arrangement for liquid syringe 200 (not shown).

In this case, when liquid syringe 200 is appropriately held by cylinder holding mechanism 120, the green pattern and the red pattern are completely matched and combined into a black pattern. If liquid syringe 200 is not properly held, the red and green colors are seen. At the same time, the shape of the combined pattern can also be seen to check whether or not cylinder member 210 is filled with liquid L.

In the above embodiment, only one position of liquid syringe 200 is defined as the position which should be located at the lower surface, but a plurality of positions may be defined as the position. For example, the liquid syringe may have a hexagonal cylinder flange and be held by the cylinder holding mechanism in a state that one side of the hexagon is positioned downward. In this case, the positions of the cylinder flange corresponding to the first, third, and fifth sides may be defined as the positions which should be located at the lower surface (not shown).

In this case, patterns are formed at the positions corresponding to the first, third, and fifth sides of the cylinder flange, while the positions corresponding to the second, fourth, and sixth sides are not provided with any pattern. For example, if the liquid syringe is held such that the first side is located at the lower surface, the fourth side with no pattern is located at the upper surface, so that it can be seen the pattern at the position corresponding to the first side.

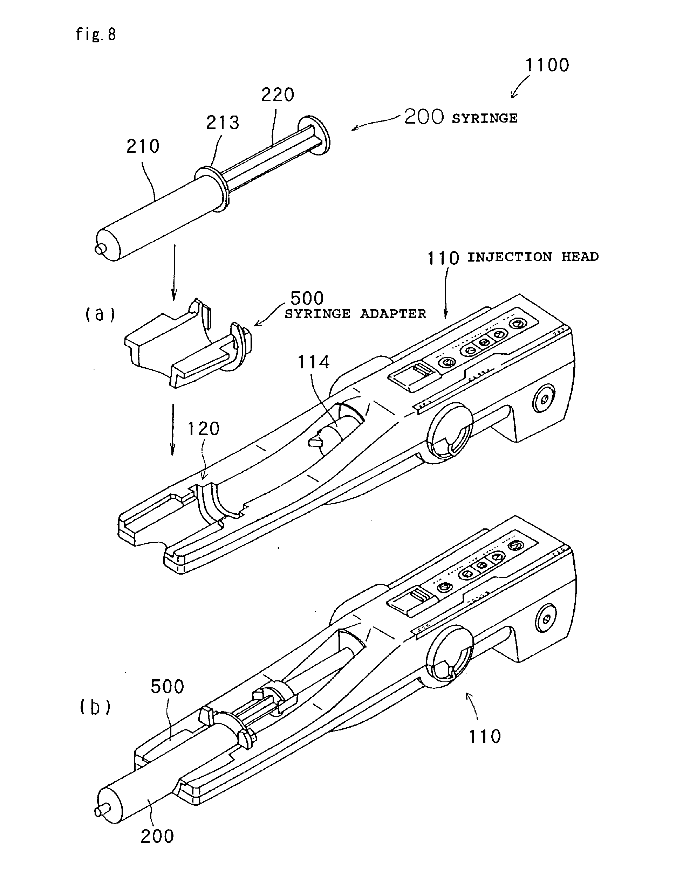

In the above embodiment, liquid syringe 200 of one type is mounted on chemical liquid injector 100 to simplify the description. In practice, a plurality of types of liquid syringes 200 are typically mounted on chemical liquid injector 100. Thus, such chemical liquid injection system 1100 will hereinafter be described in brief with reference to Fig. 8.

Chemical liquid injection system 1100 comprises chemical liquid injector 100, liquid syringes 200 of various sizes, and cylinder adapter 500 of at least one type. Cylinder adapter 500 is provided for each of liquid syringes 200 of the sizes other than the maximum size. In chemical liquid injector 100, while liquid syringe 200 of the maximum size is directly held by cylinder holding mechanism 120, liquid syringe 200 of a size other than the maximum size is held with cylinder adapter 500. Cylinder adapter 500 also has a cylinder holding mechanism (not shown) formed thereon similar to that of chemical liquid injector 100. The cylinder holding mechanism removably holds cylinder member 210 mounted from above in a state that the lower surface thereof is positioned downward.

In chemical liquid injection system 1100 of the structure described above, for example, patterns 401, 402 appearing as predetermined shapes when viewed from above through cylinder member 210 filled with transparent liquid L may be provided for the upper surface of cylinder holding mechanism 120 and the lower surface of cylinder member 210, respectively. Cylinder adapter 500 may be formed of transparent glass or resin.

In this case, patterns 401 on chemical liquid injector 100 are viewed via transparent cylinder adapter 500 and cylinder member 210, so that combined patterns 400 can be seen to check that liquid syringe 200 is appropriately held by cylinder holding mechanism 120 and that cylinder member 210 is filled with liquid L at a time.

In particular, when cylinder adapter 500 is not properly mounted on chemical liquid injector 100, or when liquid syringe 200 is not appropriately mounted on cylinder adapter 500, patterns 401, 402 are not correctly combined into patterns 400. Thus, it can be checked that cylinder adapter 500 is appropriately mounted on chemical liquid injector 100 and that liquid syringe 200 is properly mounted on cylinder adapter 500 at a time.

It is possible that cylinder adapter 500 is not appropriately mounted on chemical liquid injector 100 and liquid syringe 200 is not properly mounted on cylinder adapter 500 to result in the combination of patterns 401, 402 into patterns 400. However, in reality, this is substantially unlikely and it is unnecessary to consider that case.

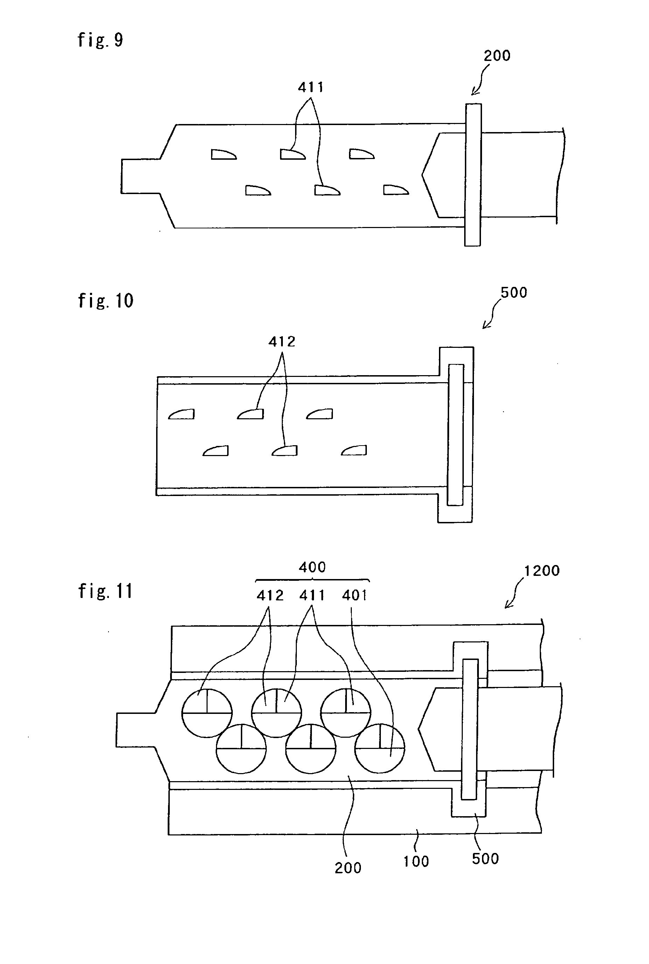

As shown in Figs. 9 to 11, in chemical liquid injection system 1200 having chemical liquid injector 100, liquid syringe 200, and transparent cylinder adapter 500 as described above, patterns 400 appearing as predetermined shapes when viewed from above via cylinder member 210 filled with transparent liquid L may be divided among the lower surface of cylinder member 210 of liquid syringe 200 of a size other than the maximum size, corresponding transparent cylinder adapter 500, and the upper surface of cylinder holding mechanism 120.

More specifically, in chemical liquid injection system 1200 as described above, patterns 401 each showing a left half of an ellipse elongated in the forward-and-rearward direction are provided for chemical liquid injector 100 as shown in Fig. 6, similarly to chemical liquid injection system 1000 described above. As shown in Fig. 9, patterns 411 each showing a rearward half of a right half of the abovementioned ellipse are provided for liquid syringe 200 of a size other than the maximum size. As shown in Fig. 10, patterns 412 each showing a forward half of the right half of the abovementioned ellipse are provided for cylinder adapter 500.

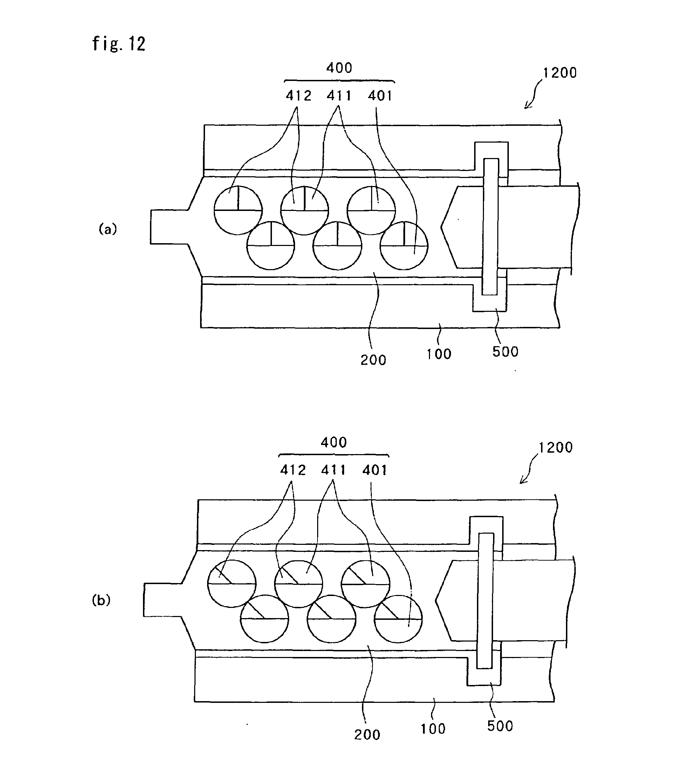

In chemical liquid injection system 1200 as described above, when cylinder member 210 of liquid syringe 200 of a size other than the maximum size is appropriately held by cylinder holding mechanism 120 via cylinder adapter 500, then patterns 401, 411, and 412 on cylinder holding mechanism 120, cylinder member 210, and cylinder adapter 500 are correctly combined into patterns 400 as shown in Fig. 11.

Thus, combined patterns 400 can be seen to check that liquid syringe 200 of the size other than the maximum size is appropriately held by cylinder holding mechanism 120 via cylinder adapter 500, and that cylinder member 210 is filled with liquid L at a time.

Patterns 412 may be provided for the interior of transparent cylinder adapter 500 as described above as well as the upper surface and the lower surface thereof. A colored part serving as pattern 412 may be embedded to extend from the upper surface to the lower surface of cylinder adapter 500.

In chemical liquid injection system 1200 as described above, patterns 402 formed by combining patterns 411, 412 on the lower surface of cylinder member 210 of liquid syringe 200 of the size other than the maximum size and on cylinder adapter 500 corresponding therewith are preferably provided for the lower surface of cylinder member 210 of liquid syringe 200 of the maximum size as shown in Fig. 7. In this case, as shown in Fig. 1, it can be checked that liquid syringe 200 of the maximum size is directly held appropriately by cylinder holding mechanism 120 and that cylinder member 210 is filled with liquid L at a time.

In chemical liquid injection system 1200 as described above, patterns 411, 412 may have different divided shapes for different sizes of liquid syringe 200 and different types of cylinder adapter 500 as shown in Fig. 12. In this case, combined patterns 400 can be seen to check that liquid syringe 200 is held appropriately by cylinder holding mechanism 120 via cylinder adapter 500, that cylinder member 210 is filled with liquid L, and that liquid syringe 200 is combined with proper cylinder adapter 500 at a time.

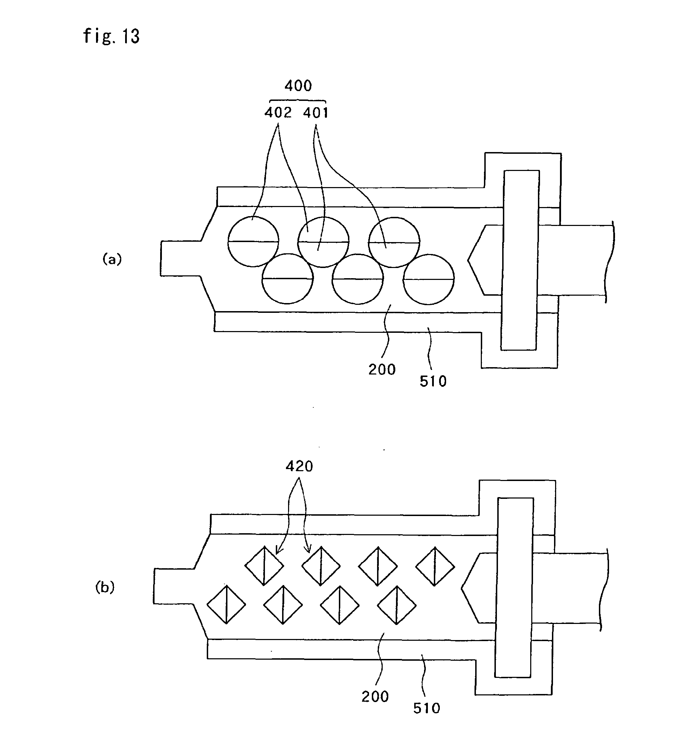

In a chemical liquid injection system (not shown) including chemical liquid injector 100, liquid syringe 200, and cylinder adapter 500, it is possible, as shown in Fig. 13(a), that cylinder adapter 510 is formed of non-transparent material and patterns 400 appearing as predetermined shapes when viewed from above through cylinder member 210 filled with transparent liquid L are divided between the lower surface of cylinder member 210 of liquid syringe 200 of a size other than the maximum size and the upper surface of corresponding cylinder adapter 510.

In this case, combined patterns 400 can be seen to check that liquid syringe 200 of the size other than the maximum size is appropriately held by cylinder adapter 510 and that cylinder member 210 is filled with liquid L at a time. Such a chemical liquid injection system cannot be checked whether or not cylinder adapter 510 is appropriately mounted on chemical liquid injector 100. However, cylinder adapter 510 is typically designed specifically for chemical liquid injector 100 and vice versa, so that cylinder adapter 510 is simply and reliably mounted and it is almost unnecessary to check whether or not cylinder adapter 510 is appropriately mounted on chemical liquid injector 100.

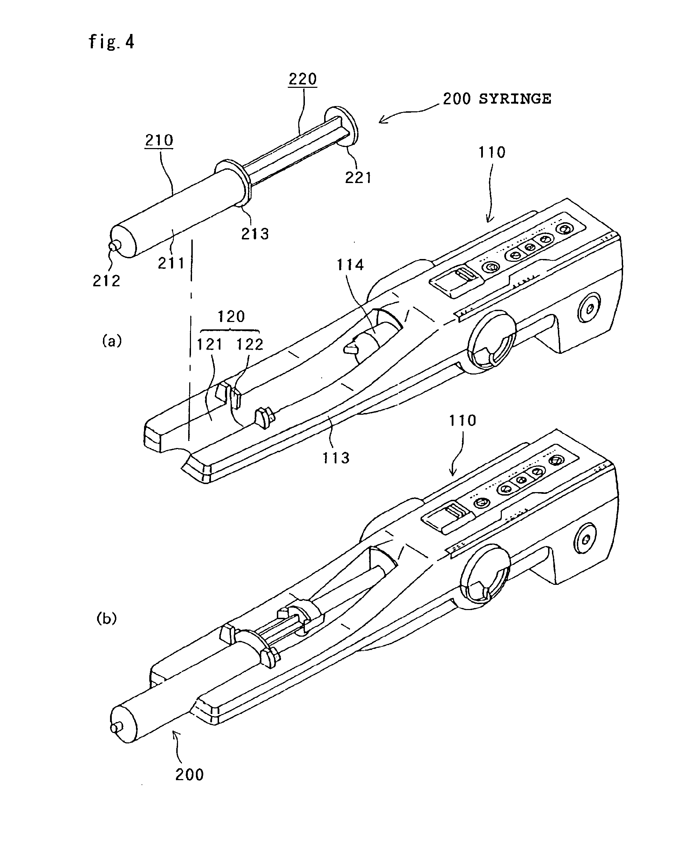

In such a chemical liquid injection system, as shown in Figs. 1 to 7, patterns 400 appearing as predetermined shapes when viewed from above through cylinder member 210 filled with liquid L are preferably divided between the lower surface of cylinder member 210 of liquid syringe 200 of the maximum size and the upper surface of cylinder holding mechanism 120.

As shown in Fig. 13, in a chemical liquid injection system (not shown) including chemical liquid injector 100, liquid syringe 200, and non-transparent cylinder adapter 510 as described above, it is possible that patterns 400, 420 have different shapes, numbers, arrangements, and colors for different sizes of liquid syringes 200 and different types of cylinder adapter 510.

In this case, combined patterns 400, 420 can be seen to check that liquid syringe 200 is appropriately held by cylinder adapter 510, that cylinder member 210 is filled with liquid L, and that liquid syringe 200 is combined with correct cylinder adapter 500 at a time.

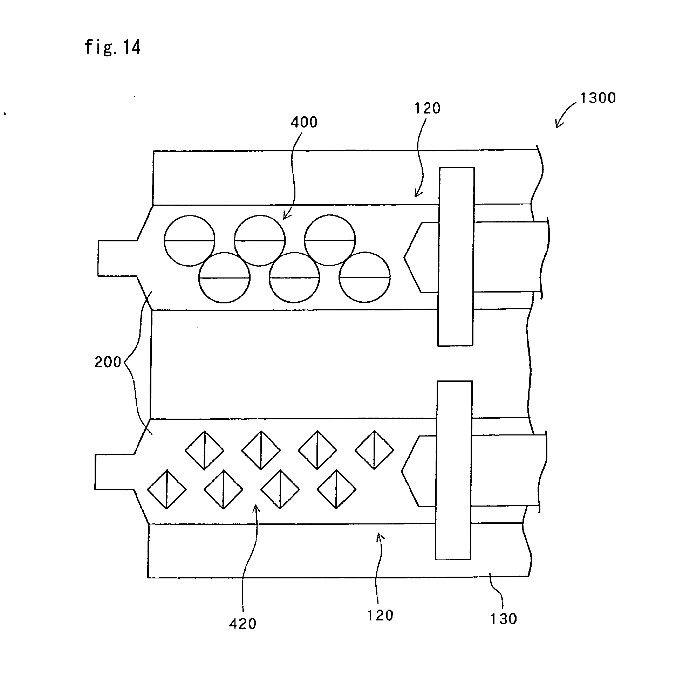

In chemical liquid injector 100 of the above embodiment, one liquid syringe 200 is held by one cylinder holding mechanism 120 and driven by one piston driving mechanism 114. For example, as shown in Fig. 14, it is possible to realize chemical liquid injection system 1300 including chemical liquid injector 130 in which a plurality of liquid syringes 200 are individually held by a plurality of cylinder holding mechanisms 120 and driven by a plurality of piston driving mechanisms 114.

In such chemical liquid injection system 1300, liquid syringe 200 filled with a contrast medium or physiological saline to be mounted is specified for each of cylinder holding mechanisms 120 and piston driving mechanisms 114 of chemical liquid injector 130. Thus, in chemical liquid injection system 1300 as described above, as shown in Fig. 14, patterns 400, 420 may have different shapes, numbers, arrangements, and colors for the plurality of cylinder holding mechanisms 120 and corresponding liquid syringes 200.

In this case, combined patterns 400, 420 can be seen to check that liquid syringe 200 is appropriately held by cylinder holding mechanism 120, that cylinder member 120 is filled with liquid L, and that liquid syringe 200 is combined with correct cylinder holding mechanism 120 at a time.

This can prevent medical malpractice such as injection of physiological saline into a patient when the operator intends to inject a contrast medium or injection of the contrast medium when the operator intends to inject the physiological saline. It goes without saying that the abovementioned various modifications may be combined without contradicting each other.

## Claims

1. A chemical liquid injection system comprising a chemical liquid injector and a liquid syringe,
wherein the liquid syringe comprises a transparent cylinder member having at least a position defined as a lower surface, and a piston member slidably inserted into the cylinder member, and
the chemical liquid injector comprises a cylinder holding mechanism removably holding the cylinder member of the liquid syringe mounted from above in a state that the lower surface is positioned downward, and a piston driving mechanism relatively moving the piston member to the held cylinder member of the liquid syringe, and
wherein a pattern appearing as a predetermined shape when it is viewed from above through the cylinder member filled with a transparent liquid is divided between the lower surface of the cylinder member and an upper surface of the cylinder holding mechanism.

2. A chemical liquid injection system comprising a chemical liquid injector, liquid syringes of various sizes, and a cylinder adapter of at least one type,
wherein each of the liquid syringes comprises a transparent cylinder member having at least a position defined as a lower surface, and a piston member slidably inserted into the cylinder member,
the cylinder adapter is formed for each of the liquid syringes of sizes other than the maximum size and removably holds the cylinder member mounted from above in a state that the lower surface is positioned downward, and
the chemical liquid injector comprises a cylinder holding mechanism removably holding the cylinder member of the liquid syringe of the maximum size mounted from above and the cylinder member of the liquid syringe of a size other than the maximum size mounted from above via the cylinder adapter in a state that the lower surface is positioned downward, and a piston driving mechanism relatively moving the piston member to the held cylinder member of the liquid syringe,
wherein, the cylinder adapter is formed transparently, and
a pattern appearing as a predetermined shape when it is viewed from above through the cylinder member filled with a transparent liquid is divided between the lower surface of the cylinder member and an upper surface of the cylinder holding mechanism.

3. A chemical liquid injection system comprising a chemical liquid injector, liquid syringes of various sizes, and a cylinder adapter of at least one type,
wherein each of the liquid syringes comprises a transparent cylinder member having at least a position defined as a lower surface, and a piston member slidably inserted into the cylinder member,
the cylinder adapter is formed for each of the liquid syringes of sizes other than the maximum size and removably holds the cylinder member mounted from above in a state that the lower surface is positioned downward, and
the chemical liquid injector comprises a cylinder holding mechanism removably holding the cylinder member of the liquid syringe of the maximum size mounted from above and the cylinder member of the liquid syringe of a size other than the maximum size mounted from above via the cylinder adapter in a state that the lower surface is positioned downward, and a piston driving mechanism relatively moving the piston member to the held cylinder member of the liquid syringe,
wherein the cylinder adapter is formed non-transparently,
a pattern appearing as a predetermined shape when it is viewed from above through the cylinder member filled with a transparent liquid is divided between the lower surface of the cylinder member of the liquid syringe of the maximum size and an upper surface of the cylinder holding mechanism, and
a pattern appearing as a predetermined shape when it is viewed from above through the cylinder member filled with a transparent liquid is divided between a lower surface of the cylinder member of the liquid syringe of a size other than the maximum size and an upper surface of the corresponding cylinder adapter.

4. A chemical liquid injection system comprising a chemical liquid injector, liquid syringes of various sizes, and a cylinder adapter of at least one type,
wherein each of the liquid syringes comprises a transparent cylinder member having at least a position defined as a lower surface, and a piston member slidably inserted into the cylinder member,
the cylinder adapter is formed for each of the liquid syringes of sizes other than the maximum size and removably holds the cylinder member mounted from above in a state that the lower surface is positioned downward, and
the chemical liquid injector comprises a cylinder holding mechanism removably holding the cylinder member of the liquid syringe of the maximum size mounted from above and the cylinder member of the liquid syringe of a size other than the maximum size mounted from above via the cylinder adapter in a state that the lower surface is positioned downward, and a piston driving mechanism relatively moving the piston member to the held cylinder member of the liquid syringe,
wherein the cylinder adapter is formed transparently,
a pattern appearing as a predetermined shape when it is viewed from above through the cylinder member filled with a transparent liquid is divided among the lower surface of the cylinder member of the liquid syringe of a size other than the maximum size, the corresponding cylinder adapter, and an upper surface of the cylinder holding mechanism, and
a pattern formed by combining the pattern on the lower surface of the cylinder member of the liquid syringe of the size other than the maximum size with the pattern on the corresponding cylinder adapter is provided for a lower surface of the cylinder member of the liquid syringe of the maximum size.

5. The chemical liquid injection system according to claim 3, wherein the liquid syringes are formed to have a plurality of sizes other than the maximum size,
the cylinder adapter is formed for each size of the liquid syringes, and
at least one of the shape, number, arrangement, and color of the pattern varies depending on the size of the liquid syringe and the type of the cylinder adapter.

6. The chemical liquid injection system according to claim 4, wherein the liquid syringes are formed to have a plurality of sizes other than the maximum size,
the cylinder adapter is formed for each size of the liquid syringes, and
the divided shape of the pattern varies depending on the size of the liquid syringe and the type of the cylinder adapter.

7. The chemical liquid injection system according to any one of claims 1 to 6, wherein the cylinder member has the divided pattern which is provided with a paint showing a predetermined color at a predetermined temperature.

8. The chemical liquid injection system according to any one of claims 1 to 7, wherein the chemical liquid injector has a plurality of the cylinder holding mechanisms and a plurality of the piston driving mechanisms,
the liquid syringe is specified for each of the plurality of cylinder holding mechanisms to be held thereby, and
at least one of the shape, number, arrangement, and color of the pattern varies among the plurality of cylinder holding mechanisms.

9. A liquid syringe of the chemical liquid injection system according to claim 1 or 2, wherein a pattern appearing as a predetermined shape when it is viewed from above through the cylinder member filled with a transparent liquid is divided between an upper surface of the cylinder holding mechanism and a lower surface of the cylinder member.

10. A liquid syringe of the maximum size of the chemical liquid injection system according to claim 3, wherein a pattern appearing as a predetermined shape when it is viewed from above through the cylinder member filled with a transparent liquid is divided between an upper surface of the cylinder holding mechanism and a lower surface of the cylinder member.

11. A liquid syringe of a size other than the maximum size of the chemical liquid injection system according to claim 3, wherein a pattern appearing as a predetermined shape when it is viewed from above through the cylinder member filled with a transparent liquid is divided between an upper surface of the corresponding cylinder adapter and a lower surface of the cylinder member.

12. The liquid syringe according to claim 11, wherein the liquid syringes of a plurality of sizes other than the maximum size are provided, and
at least one of the shape, number, arrangement, and color of the pattern varies among the plurality of sizes.

13. A liquid syringe of the maximum size of the chemical liquid injection system according to claim 4, wherein a pattern formed by combining a pattern on a lower surface of the cylinder member of the liquid syringe of a size other than the maximum size with a pattern on the corresponding cylinder adapter is provided for a lower surface of the cylinder member.

14. A liquid syringe of a size other than the maximum size of the chemical liquid injection system according to claim 4, wherein a pattern appearing as a predetermined shape when it is viewed from above through the cylinder member filled with a transparent liquid is divided among the corresponding cylinder adapter, an upper surface of the cylinder holding mechanism, and a lower surface of the cylinder member.

15. The liquid syringe according to claim 14, wherein the liquid syringes of a plurality of sizes other than the maximum size are provided, and
the divided shape of the pattern varies among the plurality of sizes.

16. The liquid syringe according to any one of claims 9 to 15, wherein the cylinder member has the pattern provided with a paint showing a predetermined color at a predetermined temperature.

17. A chemical liquid injector of the chemical liquid injection system according to claim 1 or 2, wherein a pattern appearing as a predetermined shape when it is viewed from above through the cylinder member filled with a transparent liquid is divided between a lower surface of the cylinder member and an upper surface of the cylinder holding mechanism.

18. A chemical liquid injector of the chemical liquid injection system according to claim 3, wherein a pattern appearing as a predetermined shape when it is viewed from above through the cylinder member filled with a transparent liquid is divided between a lower surface of the cylinder member of the liquid syringe of the maximum size and an upper surface of the cylinder holding mechanism.

19. A chemical liquid injector of the chemical liquid injection system according to claim 4, wherein a pattern appearing as a predetermined shape when it is viewed from above through the cylinder member filled with a transparent liquid is divided among a lower surface of the cylinder member of the liquid syringe of a size other than the maximum size, the corresponding cylinder adapter, and an upper surface of the cylinder holding mechanism.

20. The chemical liquid injector according to any one of claims 17 to 19, wherein the chemical liquid injector has a plurality of the cylinder holding mechanisms and a plurality of the piston driving mechanisms,
the liquid syringe is specified for each of the plurality of cylinder holding mechanisms to be held thereby, and
at least one of the shape, number, arrangement, and color of the pattern varies among the plurality of cylinder holding mechanisms.

21. A cylinder adapter of the chemical liquid injection system according to claim 5, wherein each of types of the cylinder adapters are provided for each of sizes of the liquid syringes other than the maximum size, and
at least one of the shape, number, arrangement, and color of the pattern varies among the types.

22. A cylinder adapter of the chemical liquid injection system according to claim 4, wherein the cylinder adapter is formed transparently, and
a pattern appearing as a predetermined shape when it is viewed from above through the cylinder member filled with a transparent liquid is divided between a lower surface of the cylinder member of the liquid syringe of a size other than the maximum size and an upper surface of the cylinder holding mechanism.

23. The cylinder adapter according to claim 22, wherein each of types of the cylinder adapters are provided for each of sizes of the liquid syringes other than the maximum size, and
the divided shape of the pattern varies among the types.
